# EUROPEAN PATENT SPECIFICATION

(11) **EP 2 834 343 B1**
(45) Date of publication and mention of the grant of the patent: **30.10.2019**
(21) Application number: 13750375.1
(22) Date of filing: 05.04.2013
(51) Int. Cl.: C12M 1/00, A01G 7/04

(54) **QUANTUM DOT LED'S TO ENHANCE GROWTH IN PHOTOSYNTHETIC ORGANISMS**
QUANTENPUNKT-LEDS ZUR FÖRDERUNG DES WACHSTUMS PHOTOSYNTHETISCHER ORGANISMEN
DIODES ÉLECTROLUMINESCENTES À BOÎTES QUANTIQUES CONÇUES POUR AMÉLIORER LA CROISSANCE DANS LES ORGANISMES PHOTOSYNTHÉTIQUES

(30) Priority: 05.04.2012 US 201261620678 P
(43) Date of publication of application: 11.02.2015
(73) Proprietor: Nanoco Technologies, Ltd., Manchester M13 9NT (GB)
(72) Inventor: PICKETT, Nigel, Manchester M20 6TR (GB); NASAANI, Imad, Manchester M20 2UL (GB); HARRIS, James, Manchester M14 6PE (GB); GRESTY, Nathalie, Chester CH3 5HG (GB)
(74) Representative: Dauncey, Mark Peter
(86) International application number: PCT/IB2013/001622
(87) International publication number: WO 2013/150388

(56) References cited:
- EP-A1- 2 499 900
- EP-A2- 2 214 218
- WO-A1-2010/053341
- WO-A1-2010/085853
- WO-A1-2010/132955
- WO-A2-2011/012714
- WO-A2-2012/060648
- US-A1- 2009 288 340
- US-A1- 2010 259 190
- US-A1- 2011 068 322
- US-A1- 2011 197 317
- US-A1- 2011 281 295
- US-A1- 2012 033 403

## Description

CROSS-REFERENCE TO RELATED APPLICATIONS: This application claims priority to provisional application serial no. 61/620,678, filed April 5, 2012.

### BACKGROUND

### 1. Field of the Invention.

This invention relates to quantum dot light-emitting diodes. More particularly, it relates to quantum dot light-emitting diodes useful for plant, algae, and bacterial growth applications.

### 2. Background.

### Light-emitting Diodes

The use of light-emitting diodes (LEDs) is becoming increasingly commonplace in everyday life. Current applications include general lighting, backlighting for liquid crystal displays, as well display screens. Light-emitting diodes are traditionally made from inorganic semiconductors, which emit at a specific wavelength, e.g. AlGaInP (red), GaP (green), ZnSe (blue). Other forms of solid state LED lighting include organic light-emitting diodes (OLEDs), wherein the emissive layer is a conjugated organic molecule such that delocalised π electrons are able to conduct through the material, and polymer light emitting diodes (PLEDs), in which the organic molecule is a polymer. Advantages of solid state lighting (SSL) over traditional incandescent lighting include superior longevity, lower energy consumption resulting from less energy loss as heat, superior robustness, durability and reliability, and faster switching times. However, SSL is expensive, and it is difficult to produce high quality white light. Several approaches to produce white light from solid-state LEDs have been explored. White light may be obtained by using three or more LEDs of differing wavelengths, *e.g*. with red, green and blue emission, producing high efficiency white light. However, this approach is very expensive and it is difficult to produce pure white light. Other approaches combine an LED emitting in the UV or blue region of the electromagnetic (EM) spectrum with a phosphor; phosphorescent materials emit at a longer wavelength than they absorb, as the absorbed radiation undergoes a Stokes shift. One such approach is to use a combination of a UV or blue LED with a number of phosphors, *e.g.* a red and a green phosphor, such as SrSi:Eu₂⁺ and SrGaS₄:Eu₂⁺, respectively. Alternatively, a blue LED and a yellow phosphor may be combined, producing a less expensive white light source, however the colour control and colour rendering index of such materials is usually poor, owing to the lack of tuneability of the LED and phosphor.

Quantum dot (QD) LED technology has been proposed as a solution to some of the limitations of traditional solid state LEDs. QDs, semiconductor nanoparticles of the order of 2-50 nm, may be tuned to emit at any wavelength from the UV to the near-IR region of the electromagnetic spectrum by controlling the particle size.

II-VI chalcogenide semiconductor nanoparticles, such as ZnS, ZnSe, CdS, CdSe and CdTe, have been extensively studied. In particular, CdSe has been widely investigated due to the tuneability of its photoluminescence over the visible range of the EM spectrum. Many reproducible, scalable syntheses are described in the prior art, from a "bottom up" approach, whereby particles are synthesised atom-by-atom, from molecules, to clusters, to particles. Such approaches use "wet chemistry" techniques.

Owing to the toxicity of Cd, it is unfavourable for commercial applications, thus attempts to find suitable alternative quantum dot semiconductors have been explored. One such candidate is the III-V semiconductor InP. Though the photoluminescence peak width is not as narrow as that of Cd-based quantum dots, InP-based semiconducting nanoparticles may be synthesised on a commercial scale with full-width half-maxima (FWHM) less than 60 nm and photoluminescence quantum yields (PLQY) greater than 90 %.

The unique properties of quantum dots arise from their dimensions. As a particle's dimensions decrease, the ratio of the surface to the interior atoms increases; the large surface area to volume ratio of nanoparticles results in surface properties having a strong influence on the properties of the material. Further, as the nanoparticle size decreases, the electronic wavefunction becomes confined to increasingly smaller dimensions, such that the properties of the nanoparticle become intermediate between those of the bulk material and individual atoms, a phenomenon known as "quantum confinement". The band gap becomes larger as the nanoparticle size is reduced, and the nanoparticles develop discrete energy levels, rather than a continuous energy band as observed in bulk semiconductors. Thus, nanoparticles emit at a higher energy than that of the bulk material. Due to Coulombic interactions, which cannot be neglected, quantum dots have higher kinetic energy than their bulk counterparts, thus a narrow band width, and the band gap increases in energy as the particle size decreases.

QDs made from a single semiconducting material passivated by an organic layer on the surface are known as "cores". Cores tend to have a relatively low quantum efficiency, since electon-hole recombination is a facilitated by defects and dangling bonds on the surface of the nanoparticles, leading to non-radiative emission. Several approaches are used to enhance the quantum efficiency. The first approach is to synthesise a "core-shell" nanoparticle, in which a "shell" layer of a wider band gap material is grown epitaxially on the surface of the core; this serves to eliminate the surface defects and dangling bonds, thus preventing non-radiative emission. Examples of core-shell materials include CdSe/ZnS and InP/ZnS. A second approach is to grow core-multishell, "quantum dot-quantum well", materials. In this system, a thin layer of a narrow band gap material is grown on the surface of a wide band gap core, then a final layer of the wide band gap material is grown on the surface of the narrower band gap shell. This approach ensures that all photoexcited carriers are confined to the narrower band gap layer, resulting in a high PLQY and improving stability. Examples include CdS/HgS/CdS and AlAs/GaAs/AlAs. A third technique is to grow a "graded shell" QD, where a compositionally-graded alloy shell is grown epitaxially on the core surface; this serves to eliminate defects resulting from strain that often arises from the lattice mismatch between the core and shell in core-shell nanoparticles. One such example is CdSe/Cd₁₋ₓZnₓSe_{1-y}S_{y}. Graded shell QDs typically have PLQYs in the region of 70-80%.

QD emission may be tuned to higher energies than the band gap of the bulk material by manipulating the particle size. Methods to alter the absorption and emission to lower energies than that of the bulk semiconductor involve doping wide band gap QDs with a transition metal to form "d dots". In one example, Pradhan and Peng describe the doping of ZnSe with Mn to tune the photoluminescence from 565 nm to 610 nm [N. Pradhan et al., J. Am. Chem. Soc., 2007, 129, 3339].

Early attempts to fabricate QD LEDs involved embedding colloidally synthesised QDs in an optically transparent LED encapsulation medium, *e.g.* silicone or acrylate. This method provides several advantages over solid-state phosphor LEDs; QDs have easily tuneable emission, strong absorption properties, and low scattering if they are monodispersed, thus these properties may be transferred to the QD LED device. However, in practise, monodispersity is difficult to achieve since current encapsulation media tend to aggregate the QDs, deteriorating their optical performance. Quantum yield may be further reduced by photo-oxidation as oxygen migrates through the encapsulation medium to the surface of the QD. Such factors present a major challenge when producing QD LEDs on a commercial scale.

By making QD LEDs the emission may be tuned right across the visible range of the EM spectrum to produce any desired colour of QD. Once encapsulated, the PL of the QD LED chip is red-shifted relative to that of the as-synthesised nanoparticles. The extent of red-shifting is dependent on the QD concentration in resin, but may range from 15-30 nm; this shift must be taken into consideration when synthesising QDs for LED applications. CFQDs may be used as an alternative to cadmium-based nanoparticles in QD LEDs, which are more favourable for commercial applications due to the undesirability of toxic cadmium. Infrared (IR) emitting nanoparticles such as CdTe, PbS and PbSe may be used to tune LED emission to the IR region of the EM spectrum.

U.S. Pub. No. 2010/0123155 discloses the preparation of "QD-beads", in which QDs are encapsulated into microbeads comprising an optically transparent medium; the QD-beads are then embedded in a host LED encapsulation medium [Bead diameters may range from 20 nm to 0.5 mm, which may be used to control the viscosity of the QD-bead ink and the resulting properties, such as ink flow, drying, and adhesion to a substrate. QD-beads offer enhanced stability to mechanical and thermal processing relative to "bare" QDs, as well as improved stability to moisture, air, and photo-oxidation, allowing potential for processing in air which could reduce manufacturing costs. By encapsulating the QDs into beads, they are also protected from the potentially damaging chemical environment of the encapsulation medium. Microbead encapsulation also serves to eliminate the agglomeration that is detrimental to the optical performance of bare QDs in LEDs. Since the surface of the nanoparticles is not drastically disrupted or modified, the QDs retain their electronic properties when encapsulated in microbeads, allowing tight control over the QD-bead ink specification.

The QD LED structures described herein overcome some of the shortcomings of those described in the prior art.

### Photosynthesis

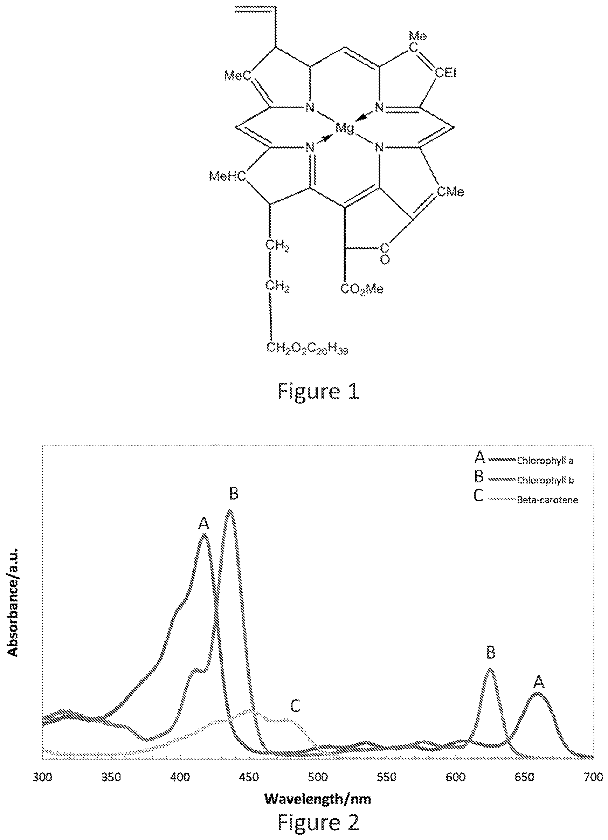

Photosynthesis, the process by which plants, algae and certain bacteria absorb sunlight to produce their own energy, is fundamentally dependent on light absorption by the green pigment chlorophyll. In plants, absorption predominates in the blue and red ends of the electromagnetic spectrum; blue absorption promotes vegetative growth, while red absorption promotes flowering and budding. Chlorophyll exists in a number of forms, predominantly chlorophyll *a* (Figure 1). All plants contain chlorophyll *a* for photosynthesis. In addition, accessory pigments absorb energy that is not absorbed by chlorophyll *a;* these include chlorophyll *b* (and *c*, *d* and *f* found in algae and bacteria), and carotenoids (xanthophylls and carotenes).

Studies into the absorption spectra of chlorophylls have shown that their molecular environment has a significant influence on their spectroscopic properties. Factors including solution concentration and temperature, microcrystallisation, and adsorption of chlorophyll onto a film have been shown to alter its absorption spectrum. *In situ,* chlorophyll is present in high concentrations and in a partially ordered state [R. Livingston, Q. Rev. Chem. Soc., 1960, 14, 174]. Even so, the majority of studies into the absorption of chlorophyll are carried out in dilute solutions.

The absorption spectrum of chlorophyll a in methanol is shown in Figure 2 A. Chlorophyll a has two absorption maxima, one around 420 nm and another around 660 nm. The relative intensities of blue to red absorption are approximately 3:1 in ether [D. Houssier and K. Sauer, J. Am. Chem. Soc., 1970, 92, 779].

Chlorophyll *b* has two absorption maxima, as shown in the absorption spectrum in diethyl ether (Figure 2 B); it absorbs strongly in the blue at around 460 nm, with a shoulder around 435 nm, and in the red with an absorption maximum around 645 nm. The relative absorption intensities in the blue and the red are solvent dependent, but are almost 3:1 in dioxane and acetone [A. Pfarrherr et al., J. Photochem. Photobiol. B: Biol., 1991, 9, 35].

Non-chlorophyll accessory pigments absorb light not absorbed by chlorophyll, and may also work as antioxidants. For example, β-carotene has broad blue absorption (Figure 2 C). Xanthophylls, such as lutein and zeaxanthin, also absorb in the blue. Plants with coloured leaves, such as red cabbage that has dark red-purple leaves, contain high concentrations of other pigments relative to chlorophyll; high concentrations of anthocyanins give red or purple leaves, while carotenoids give yellow leaves. Anthocyanins are not directly involved in photosynthesis. A high proportion of other pigments relative to chlorophyll in the leaves of a plant reduces the rate of photosynthesis, thus higher light intensities would be beneficial to their growth.

The arrangement of proteins, pigments and cofactors in complexes that are used during photosynthesis are known as "photosynthetic reaction centres" or photosystems. In these complexes, chlorophyll may be bound to other pigments, which may alter its absorption properties. For example, in Photosystem I, there is a long wavelength absorption band around 705 nm, which is also observed for chlorophyll dimers, as well as chlorophyll-phæophytin aggregates (phæophytin has the structure of chlorophyll a without the Mg²⁺ centre), chlorophyll-lutein and chlorophyll-zeaxanthin complexes [S.S. Brody et al., J. Chem. Soc., Faraday Trans. 2, 1986, 82, 2245]. Thus, the absorption properties of a plant during photosynthesis are not only dependent on the absorption spectra of the individual pigments, but also the complexation of pigments present in its leaves.

In addition to the pigments involved in photosynthesis, other pigments are present in plants to sustain their development and growth. Processes such as phototropism, whereby plants adjust their position in response to a light stimulus, and photoperiodicity, which signals the seasonal day-night pattern to a plant, are also controlled by light-absorbing pigments. Photoperiodicity is regulated by phytochrome and cryptochrome. Phytochrome absorption occurs in the red and far-red regions of the EM spectrum. Cryptochrome absorbs ultraviolet light.

Algae also contain chlorophylls for photosynthesis. Green algae contain chlorophylls a and b, which red algae additionally contain phycobilin pigments that absorb red, orange, yellow and green light. Cyanobacteria, also referred to as blue-green algae, use phycocyanin to absorb orange-red light (typically around 620 nm) for photosynthesis. Some bacteria contain bacteriochlorophylls, which absorb IR radiation to produce energy during photosynthesis. In contrast to photosynthesis in plants, photosynthesis using bacteriochlorophylls does not produce oxygen.

### Artificial Lighting to Stimulate Plant Growth

In recent years, artificial lighting has featured increasingly in the growth of plants. The invention of plant factories has enabled crops and flowers to be grown in countries where the climate and environment are not naturally suited to their growth. Plant factories enable factors such as light, temperature, humidity, CO2 concentration and soil to be manipulated to suit the requirements of the plants to be grown, without the need for pesticides or fungicides. In plant factories, growth is not affected by the seasons, thus seasonal crops may be grown all year round. Crop yields are unaffected by changing weather patterns and unpredictable extreme weather conditions, such as flooding, drought and high winds. In addition, factories enable plants to be grown on different tiers, maximising the space available, which would not be possible on traditional farmland. The ability to produce crops where they would not naturally thrive has potential environmental advantages; seasonal or exotic crops may be grown locally all year round, reducing import and transportation costs along with their associated pollutant and CO2 emissions. As a further advantage, artificial lighting may be tuned to optimise plant growth by selectively matching the emission of the light sources to the absorption spectra of the chlorophyll and accessory pigments in the plants. The unrequired parts of the EM spectrum need not be emitted, thus maximising the energy efficiency of the process.

Artificial lighting may also be used to promote grass growth during the winter months. For example, in the sports industry areas of turf have to be reseeded between fixtures, such as goal mouths on football pitches. In the northern hemisphere, during the winter months the lighting conditions may be insufficient to promote grass growth in the short timeframe between matches, thus there is a need for portable artificial lighting devices that may be used to stimulate grass growth.

Originally, broad-spectrum artificial light sources were used in plant factories. Artificial broad-spectrum lighting is inefficient for promoting horticultural growth. Since absorption does not take place over the entire electromagnetic spectrum during photosynthesis much energy is wasted in producing broad spectrum light. Further, considerable amounts of energy are dissipated as heat, leading to further energy wastage and potential damage to the plants. Fluorescent lamps emitting in the red and the blue have been proposed as an alternative, however frequent use may lead to filament damage, thus short lifetimes. Additionally, their mercury exciter elements may leak toxic mercury if the bulb is broken. High-pressure sodium lamps have also been investigated; these emit strongly in the red region of the EM spectrum, but not in the blue. LEDs, with their narrow emission at specific wavelengths, have been proposed as advantageous alternatives. LEDs may be tuned to emit at wavelengths with peak maxima that coincide with the absorption spectrum of chlorophyll, and the ratio of blue to red LEDs may be balanced to match the absorption intensities of blue and red light by chlorophyll. Despite the lower energy consumption, solid state LED lighting is still very expensive on a large scale, resulting in a high initial investment cost to install SSL in plant factories.

Early proposals for the use of LED lighting for horticultural growth applications in the late 1980s and early 1990s was mainly reliant on red LED lighting, since high-efficiency blue LEDs had not yet been developed. U.S. Patent No. 5,012,609 describes a system of LEDs emitting at 620-680 nm to match the absorption spectrum of chlorophyll, with lower intensity output from LEDs emitting between 700-760 nm to meet the photomorphogenic (a change in form in response to light) needs of the plant. LED or neon lighting in the 400-500 nm range is also suggested to support the plant's photomorphogenic and phototropic requirements. Other researchers found that red LED lighting around 660 nm, used in conjunction with blue fluorescent lamps, could be used to grow lettuce with equivalent characteristics to lettuce plants grown under cool-white fluorescent and incandescent lamps [R.J. Bula et al., HortScience, 1991, 26, 203]. Using red LED lighting without any blue light resulted in abnormalities in the growth of the lettuce [M.E. Hoenecke et al., HortScience, 1992, 27, 427].

Since the invention of high-efficiency blue solid state LED lighting, developments have been made on the structural design of lighting systems and the arrangement of the LEDs to optimise plant growth.

U.S. Patent No. 6,554,450 to Fang et al. describes an LED lighting arrangement tailored towards tissue culture growth and the development of young plants. The LEDs are detachable and the relative light intensities of the LEDs are adjustable. The device operates on a timer whereby the plants are illuminated for 16 hours, then kept in the dark for 8 hours each day.

U.S. Pub. No. 2006/0006820 discloses a horticultural lighting system using solid state LEDs that emit not only at the wavelengths required for photosynthesis, but may also be controlled to emit at wavelengths to stimulate other growth processed within the plant. For example, emission at 290-320 nm and 380 nm may stimulate cryptochrome, which regulates the circadian rhythm of plants, along with their directional growth in response to light. 705-745 nm emission is used for phytochrome stimulation, which regulates the growth cycle of plants. Disadvantageously, the lighting system uses third generation solid state LEDs, which dissipate a considerable amount of heat.

U.S. Pub. No. 2010/0259190 describes lighting fixtures with a single blue LED tuned with phosphors to emit at multiple wavelengths. The device uses a solid state LED emitting in the 300-500 nm range, with a number of phosphors in the 350-550 nm and optionally in the 600-800 nm and/or 350-800 nm ranges to adjust the emission peak maxima to match the photosynthetic requirements of the plants. The lighting fixture includes a dial to enable adjustment of the peak wavelength.

Current solid state LED research into horticultural lighting focusses on developing high-quality LEDs that emit at similar wavelengths to the absorption maxima of chlorophyll, i.e. 455 nm and 660 nm. Red LEDs, particularly those emitting around 660 nm, tend to have lower efficiency than blue LEDs. Leaders in the LED lighting market, such as Osram Opto Semiconductors, Philips, Everlight and Showa Denko, have all studied LED lighting to meet the criteria of plant factories. Attention has been focussed on improving the efficiency of LED emission at 660 nm, as well as enhancing LED lifetimes, and optimising the lighting arrangements to promote growth while maximising the utilisation of space.

U.S. Pub. No. 2009/0288340 describes an LED lighting system comprising blue and red LEDs with a cooling system to conduct heat away from the LEDs, including a fan to vent heat from the outside of the housing unit, to avoid the detrimental effects of heat from the light source on plant development. The emissive intensities of the blue and red LEDs may be tuned independently, or substituted with a white LED, to meet the needs of the growing plants.

WO 2010/066042 A1 describes a red-green-blue (RGB) LED lighting system in which light from pre-existing RGB LED packages is emitted from a geometrically common point of origin. It is claimed that this is advantageous over conventional horticultural LED lighting in which the LEDs are discretely located, since it eliminates colour hot spots and colour specific shadows.

U.S. Pub. No. 2011/0115385 describes a system of 24 red (660 nm), 12 orange (612 nm) and 2 blue (470 nm) solid state LEDs, in a circular arrangement, to optimise horticultural growth. This system is designed such that the beams of light from the LEDs may be redirected as plants grow or are relocated in the factory, improving the long-term efficiency of the lighting system over the lifetime of the plants in comparison to previous inventions in the prior art.

U.S. Pub. No. 2010/0031564 describes a plant growth device using OLEDs. The device comprises a rack, for tiered growth, with OLEDs as "grow lights" and "control lights". The grow lights emit in the 400-500 nm and 600-700 nm ranges with 10-20 % and 80-90 % of the relative light intensity, respectively, to support the photosynthetic needs of the plants, while the control lights may be used to steer growth, for example, high intensity blue light produces large plants, while lower intensity blue light results in smaller, more compact plants. The OLEDs are used in the place of conventional SSL as they are more easily tuned.

WO 2011/016521 A1 describes an AlGaInP-based LED that emits at 660 nm, beyond the normal emission range of AlGaInP, with an external quantum efficiency around three times that of a conventional red LED such that it is comparable to that of blue LEDs.

Commercial LED systems to enhance horticultural growth have been developed by Everlight, Osram and Philips.

In October 2010, Everlight launched their "GL-Flora" lighting fixture, specifically designed to promote plant growth. The emission wavelengths and LED ratios are designed to provide a homogeneous light distribution that promotes growth while controlling germination and flowering.

In 2010, Osram announced two commercial partnerships for their LEDs for horticultural applications. The first project, with the Danish horticultural LED lighting company Fionica Lighting A/S (September 2010), uses their "Golden Dragon Plus" and "Oslom SSL" LEDs, both of which emit at 660 nm with approximately 37-40 % efficiency and a lifetime of around 100,000 hours. The "Golden Dragon Plus" system has a 170° beam angle that is useful in reflector systems for lighting large areas with red and blue emission at 660 nm and 449 nm, respectively, while the 80° beam angle of the "Oslon SSL" system allows the LEDs to be packed closely to one another for stacked multilayer arrangements that are typically used to grow salad plants. The "Oslon SSL" LEDs emit at 660 nm and 452 nm. In November 2010, Osram announced the use of their "TopLED" system for horticultural lighting by the Finnish greenhouse lighting company Netled Oy. This system, with its curtain structure, is expected to reduce energy consumption by 60% relative to high pressure sodium lighting systems.

In 2009, Philips launched their "GreenPower" LED lighting range. The GreenPower LED module is a system of 5 LEDs encased in a waterproof carrier. The customer may choose from blue, red and far-red LEDs, which may be changed during the growth cycle of the plants to meet changing light intensity and colour ratio requirements. For multilayer applications, including tissue culture and plant transportation, they have devised the GreenPower LED string, whereby blue or red LEDs are arranged in a flexible chain. Initial testing for tissue culture applications suggested a 50-80 % energy saving.

Many of the LED lighting systems described in the prior art employ the use of multiple coloured solid state LEDs. Not only is there a high cost associated with purchasing multiple LEDs of different colours, there is the added cost of the increase in circuitry required for each colour of LED.

### Artificial Lighting to Promote the Growth of Algae and Bacteria

In the search for biofuels to replace fossil fuels, algae have become important candidates as an alternative fuel source. In comparison to crops, the oil and fuel yields from algae may be 10-100 times higher, with the species botryoccus braunii producing up to 86% of its dry weight in hydrocarbons. Microalgae have high oil content, thus produce high mass to fuel conversion, however cost-effective cultivation may be difficult. Macroalgae contain lower concentrations of carbohydrates and lipids, however are more inexpensively cultivated. Trans-esterification of the oil from algae may be used to generate biodiesel, while methanol and ethanol may be produced from its anaerobic digestion and fermentation, respectively. Emissions from biodiesel are reportedly around 75-80% lower than those from regular diesel. Direct combustion of algae biomass may be used generate heat and electricity.

Algae may be used in wastewater management, since they are able to grow in sewage and wastewater; they may be used to remove both toxic substances and nutrients from water, which may be advantageous in the water purification process.

Since algae require 1.8 times their mass in CO₂ for photosynthesis, they may be used to reduce CO₂ emissions from factories and power plants. Using algae to capture and convert CO₂ to O₂ during photosynthesis is a novel way for companies to reduce their CO₂ emissions to comply with the Kyoto Protocol, in which 37 countries have pledged to reduce their greenhouse gas emissions by 5.2 % relative to their 1990 levels [Kyoto Protocol to the United Nations Framework Convention on Climate Change, 1998].

Algae are rich in many nutrients such as B-vitamins and iodine, and pigments. Thus they have been proposed for nutraceutical purposes, as natural dyes, and for use in organic fertilisers.

Biodegradable plastics may be fabricated using algae. Not only do biodegradable plastics fully decompose, they may also require lower processing temperatures than traditional plastics, offering energy saving and reduced greenhouse gas emissions during manufacture. The US company Cereplast has developed an algae bioplastic that has been launched in commercial products such as hair accessories by "The Barrette Company". The Indian company BNT Force Biodegradable Polymers has patented a method for producing biodegradable plastics incorporating blue-green algae [US 8,026,301 B2, 2011]. The plastic degrades in 6-36 months, without leaving behind any toxic residues.

Bacteria are used in many industries, from fermentation to pharmaceuticals, bioremediation, fibre retting and pest control. In certain applications, bacteria may provide an environmentally friendly alternative to chemical treatments. When such applications utilize photosynthetic bacteria, artificial light to stimulate their growth could be beneficial to accelerate treatment processing.

Bioremediation is the removal of pollutants using microorganism metabolism. Certain bacteria may ingest hydrocarbons, which may be used to clean up oil spills or areas contaminated with heavy metals. For example, *Rhodobacter sphaeroides,* a purple non-sulphur bacterium that contains the pigments bacteriochlorophyll a and bacteriophæophytin a, has been studied for bioremedial use in areas polluted with metal contaminants [L. Giotta et al., Chemosphere, 2006, 62, 1490]. The bacterium shows a high resistance towards MoO₄²⁻, Co²⁺ and the ability to convert CrO₄²⁻ to Cr³⁺, which other bacteria are unable to achieve. *R. sphaeroides* has a strong absorption band around 865 nm. The present invention may be applied, for instance, following a spillage of contaminants when rapid bacteria are employed to assist in the decontamination process; artificial lighting targeted towards photosynthetic bacterial growth may help to accelerate the clean-up process.

Photobioreactors are routinely used to grow algae, particularly for biofuel applications. They may be advantageous over open pond systems, since they allow control over factors such as temperature and lighting, as well as providing protection from competing or contaminating species. Studies have shown that increased light intensity may increase the oil production from algae, thus a lighting system specifically designed to enhance photosynthesis in algae may be an advantageous component to a photobioreactor. In the prior art, a small number of patent applications have been filed and/or granted relating to the promotion of algaeal and microbial growth. Most relate to incubation systems that comprise components to enhance photosynthetic growth, e.g. water, nutrients, CO2 and light sources, however little focus has been paid to the lighting sources.

U.S. Patent No. 7,824,904 highlights the flaw in pre-existing photobioreactors and open ponds, in which light is only accessible to algae growing near the surface, since light is unable to penetrate lower down in the chamber. The patent describes a photobioreactor with rotating and/or oscillating lighting and mixing systems to distribute light throughout the tank. A light source located outside the tank is coupled to a light emitting device mounted on blades or a rotating mixing system to provide light to all areas of the tank.

U.S. Pub. No. 2010/0297739 describes apparatus constructed of a light-reflecting material, such as aluminium or stainless steel, are proposed in conjunction with a light source in a vessel designed to cultivate photosynthetic organisms for renewable energy applications. The application proposes lens-based systems to harness natural light, such as optical wave guides and Fresnel lenses.

U.S. Patent No. 8,017,377 describes a vessel with a heat exchanger, CO2 injection facilities and a continuous lighting source to promote microalgael growth for lipid production. The patent states that any light source capable of emitting 450-475 nm and 530-675 nm light may be utilised.

### BRIEF SUMMARY

Disclosed herein are quantum dot light-emitting diode based lighting systems to promote and optimise plant growth for the agricultural and horticultural industries, to promote the growth of algae for applications including biofuels and waste management, and to enhance photosynthetic bacterial growth for bioremediation purposes. Also disclosed are methods of fabricating QD LEDs for plant, algael and photosynthetic bacterial growth applications. Herein, a number of methods of fabricating LEDs from QD material are described, each comprising a blue or UV solid state LED as the primary light source in conjunction with one or more QD-based architectures as a secondary light source.

QD LED lighting provides a less expensive alternative to solid state LED lighting, moreover only one set of electronics is required as only one colour LED chip is needed, since the other wavelengths are obtained by the down-conversion of light. Using QDs, the emission wavelength of the LED may be easily tuned to match the absorption spectra of the chlorophylls and accessory pigments in plants and algae, as well as bacteriochlorophyll in photosynthetic bacteria. Emission at 660 nm is more easily achieved using QD LEDs than solid state LEDs. The photoluminescence (PL) full-width half-maxima (FWHM) of QDs may be tuned to match the absorption spectra of chlorophylls, accessory pigments and bacteriochlorophyll in photosynthetic organisms. QDs may be synthesised with a high photoluminescence quantum yield in the red region of the EM spectrum; this overcomes the issue with solid state LED lighting for which emission is much more intense in the blue than the red region of the EM spectrum. The lifetime of QD LEDs is in the region of 25,000-50,000 hours, which is far superior to incandescent bulbs (500 hour typical lifetime) and compact fluorescent lamps (3000 hour typical lifetime). QD LEDs have high energy efficiency, typically 30-70 lumens per Watt, compared to 10-18 lm/W for incandescent bulbs and 35-60 lm/W for fluorescent lamps. QD LEDs give off less heat, which may potentially damage plants or other photosynthetic organisms, than other artificial light sources. Certain embodiments may be used to promote the growth of algae and photosynthetic bacteria. In comparison to the lighting systems described in the prior art, QD LEDs may provide a higher light intensity and emit at wavelengths more targeted to the promotion of growth of bacteria or algae, thus minimising energy wastage. In addition, the low heat dissipation from QD LEDs may not influence the incubation temperature within a photobioreactor. Using quantum dot LEDs, IR-emitting quantum dots may be used to fabricate IR-emitting LEDs, which may be applicable to bacterial growth applications. For bioremediation applications, for which bacteria are specifically chosen with resistance to heavy metal toxicity, the unfavourability of using small quantities of heavy metals in the IR-emitting QDs (which would provide a minimal risk of contamination through exposure once encapsulated in the LED device) may be negated by the greater risk of heavy metal exposure from the contamination site.

The disclosed systems have several advantages over systems that use different coloured LEDs. In the disclosed systems, all colours of the emitted light originate from a single location, avoiding colour hot spots. Using a single solid state LED light source with QDs to tune the emission also reduces cost associated with the increased circuitry required for multiple coloured solid-state LED arrangements.

### BRIEF DESCRIPTION OF THE SEVERAL VIEWS OF THE DRAWING(S)

Figure 1 shows the molecular structure of chlorophyll a.
Figure 2 is a UV-vis absorption spectra of chlorophyll a in methanol (A), chlorophyll b in diethyl ether (B) and β-carotene in hexane (C). All data were taken from the photochemCAD database [H. Du et al., Photochem. & Photobiol., 1998, 68, 141]. N.B. the absorption spectra are solvent dependent.
Figure 3 is a schematic diagram of a QD LED, wherein red and blue QDs are embedded in beads and illuminated with a UV LED primary light source.
Figure 4 is a schematic diagram of a QD LED, wherein red and blue QDs are embedded in separate beads and illuminated by a primary UV light source.
Figure 5 is a diagram illustrated the structure of a QD LED chip. The QDs in resin are loaded into the LED case, which is illuminated from below by a UV or blue solid state LED.
Figure 6 is a schematic diagram showing a remote phosphor architecture. In the illustrated example, a blue solid state LED illuminates a red QD phosphor from below, which emits both blue and red light.
Figure 7 illustrates preparation of a QD phosphor sheet: (A) QD ink is drop cast on the region between the spacers of the PET substrate (B). QD ink is distributed uniformly between the spacers by using a glass slide.
Figure 8 is a diagram showing an arrangement for illumination of a QD phosphor by a solid state LED (in the illustrated example emitting UV light) positioned to the side of the phosphor sheet. In the example, UV light from the LED passes through the QD phosphor, while down-converted blue and red light from the QD phosphor is emitted towards the plant below.
Figure 9 is an illustration showing a petri-dish filled with agar (as a culture for growing photosynthetic bacteria) embedded with IR-emitting QD beads. The petri-dish is illuminated by UV or blue solid state LEDs.

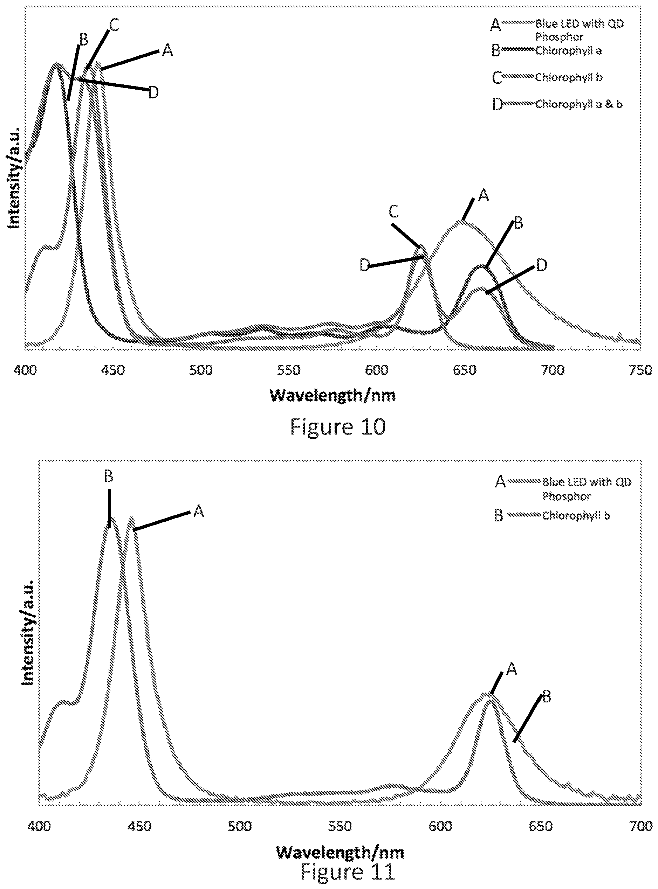

Figure 10 is a plot showing the emission spectrum of a remote phosphor QD LED comprising a blue solid state LED (22 mW) as the primary light source and a red quantum dot (InP/ZnS) silicone resin (emitting at 648 nm with FWHM of 59 nm) at a concentration of 20 ODs per 10 mmol of solution as a secondary light source (A), alongside the absorption spectra of chlorophyll a (B), chlorophyll b (C), and the combined chlorophyll a and b spectra (D). The plot shows that the emission maxima and relative peak intensities of the QD-phosphor LED are well matched to the absorption spectra of chlorophyll a and b.
Figure 11 is a plot showing the emission spectrum of a remote phosphor QD LED comprising a blue solid state LED as the primary light source and a red quantum dot (CdSe/CdS/CdZnS/ZnS) silicone resin (emitting at 625 nm with FWHM of 35 nm) as a secondary light source (A), alongside the absorption spectrum of chlorophyll *b* (B). The plot shows that the emission spectrum of the QD-phosphor LED is well matched to the absorption spectrum of chlorophyll *b*.
Figure 12 is a diagram showing an arched (or caged) lighting arrangement that allows blue and red QD LEDs to illuminate a plant from many directions, to promote uniform plant growth.
Figure 13 is an illustration of a photobioreactor for growing algae. A red QD phosphor sheet shaped with finger-like projections is immersed in the photobioreactor, which is illuminated from above (and/or below) with blue solid state LEDs, emitting blue and red light throughout the reactor.
Figure 18 is an illustration of a photobioreactor fabricated from a transparent material encapsulating a red QD phosphor. The photobioreactor is illuminated with external blue solid state LEDs, emitting both red and blue light into the reactor.
Figure 19 is a photograph of cane-supported plants. QD-embedded transparent canes may be used to support and stimulate the growth of certain plants.
Figure 20 is a photograph of shelving units made from a clear material. Clear shelving material with a QD phosphor coating, or shelving from an injection-moulded QD material (as described in U.S. Pub. No. 2009/0246900 A1 "Shaped Articles Comprising Semiconductor Nanocrystals and Methods of Making and Using Same") may be used to stimulate the growth of certain plants.

### DETAILED DESCRIPTION OF THE INVENTION

Disclosed is a method of fabricating LEDs optimised for promoting growth of photosynthetic organisms, using a blue or UV solid state LED, with tuned emission using red (and/or other colours, as required, of) quantum dots, to emit light of the correct wavelengths and relative intensities to enhance photosynthesis. QD LEDs may be produced to emit from the blue to the UV region of the electromagnetic spectrum to match the absorption characteristics of chlorophylls and other pigments present in photosynthetic organisms to promote and support their growth.

Plant factory lighting described in the prior art utilises incandescent, fluorescent or solid state LED lighting, while patented photobioreactors focus little attention on the lighting source. The solid state LEDs described in the prior art are relatively expensive to produce. QD LEDs provide a less expensive alternative, since a very small amount of semiconductor material is required to produce bright, stable emission. The lifetime of QD LEDs is in the region of 25,000-50,000 hours, which is far superior to incandescent bulbs (500 hour typical lifetime) and compact fluorescent lamps (3000 hour typical lifetime). Further, QD LEDs have high energy efficiency, typically 30-70 lumens per Watt, compared to 10-18 lm/W for incandescent bulbs and 35-60 lm/W for fluorescent lamps. Thus, though the initial installation cost of QD LED lighting in plant factories or photobioreactors may be higher than that using incandescent or fluorescent bulbs, the superior longevity and efficiency of QD LEDs make them a favourable long-term investment.

Stable, intense emission at 660 nm, which corresponds to the red absorption maximum of chlorophyll a, is difficult to achieve with solid state LEDs, for which the emission is determined by the band gap of the semiconducting material. Solid state materials emitting at 660 nm are typically limited to AlGaInP-based semiconductors. Using QD LEDs, emission at 660 nm is far more easily achieved, since the emission wavelength may be tuned by changing the nanoparticle size. Thus, red emission may be obtained using a variety of materials, including CdSe/ZnS and InP/ZnS core-shell nanoparticles.

Because of the facile wavelength tuneability of QDs, QD LEDs may be easily modified to suit a variety of different photosynthetic organisms, including plant species, algae and photosynthetic bacteria. Simple modifications to the synthesis of the QDs may be employed to alter the PL emission, without having to change the inherent semiconducting material or the reagents used for the synthesis. Wavelength tuneability is far more easily achieved using QD LEDs than solid state LEDs. Thus, bespoke QD LED systems may more easily be produced by selecting the desired combination of QD materials from a given range, to be incorporated into the QD LED device.

Narrow emission FWHM (less than 40 nm) is most easily achieved using cadmium-based QDs, however core-shell CFQD material with FWHM less than 60 nm may be synthesised. Further, it is possible to tune the FWHM of QD material to match the absorption spectra of various photosynthetic pigments. For instance, the absorption spectrum of chlorophyll b shows narrower peak widths than that of chlorophyll a; using QDs it is possible to fabricate LEDs that emit not just at the absorption maxima of chlorophyll a and b, but also with similar FWHM and relative intensities. IR-emitting QDs, such as CdTe, PbS and PbSe could be used to produce QD LEDs emitting in the IR with emission characteristics to coincide with the absorption spectra of bacteriochlorophylls.

One of the drawbacks of the lighting systems described in the prior art is the amount of energy emitted as heat. High power solid state LEDs also give off a relatively large amount of heat in comparison to QD LEDs. Thus, for plant factory settings where temperature control is important to plant development, QD LEDs with their low heat emission are ideal. Using QD LED lighting, systems to cool the lighting device, as described in the prior art, are not required. This reduces the complexity of the lighting apparatus, keeping the cost low.

Emission of multiple wavelengths of light from a single geometric location is preferable to emission of each wavelength from a discrete location, since it eliminates colour hot spots and colour specific shadows that may lead to non-uniformity in growth. An advantage of the systems described herein is that multiple wavelengths of light may be emitted from a single direction using a single solid state LED source. Using QDs to tune the emission eliminates the need for multiple solid state LEDs that may be costly. Further, the present invention requires less circuitry than lighting arrangements using multiple solid state LEDs, which require separate circuitry for each colour LED. Not only does this reduce the cost associated with the circuitry, it is also particularly advantageous for portable devices, which could be used, for example, for stimulating grass growth for reseeding sports pitches, or for domestic grow lights.

The QD LED devices described herein may be produced in a variety of shapes and sizes, from small LED chips to QD phosphor sheets that may be fabricated in any shape or size and could be printed on flexible substrates. This enables their use in a range of applications.

The QDs used herein are optimally made from core-shell semiconductor nanoparticles. For example, the core material may be made from:
II-VI compounds including a first element from group 12 (II) of the periodic table and a second element from group 16 (VI) of the periodic table, as well as ternary and quaternary materials including, but not restricted to: CdSe, CdTe, ZnS, ZnSe, ZnTe, ZnO, HgS, HgSe, HgTe, CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, CdZnSeS, CdZnSeTe, CdHgSeS, CdHgSeTe, CdHgSTe, HgZnSeS, HgZnSeTe.
II-V compounds incorporating a first element from group 12 of the periodic table and a second element from group 15 of the periodic table, and also including ternary and quaternary materials and doped materials. Nanoparticle material includes, but is not restricted to: Zn₃P₂, Zn₃As₂, Cd₃P₂, Cd₃As₂, Cd₃N₂, Zn₃N₂.
III-V compounds including a first element from group 13 (III) of the periodic table and a second element from group 15 (V) of the periodic table, as well as ternary and quaternary materials. Examples of nanoparticle core materials include, but are not restricted to: BP, AlP, AlAs, AlSb, GaN, GaP, GaAs, GaSb, InN, InP, InAs, InSb, AlN, BN, GaNP, GaNAs, InNP, InNAs, GAInPAs, GaAlPAs, GaAlPSb, GaInNSb, InAlNSb, InAlPAs, InAlPSb.
III-VI compounds including a first element from group 13 of the periodic table and a second element from group 16 of the periodic table and also including ternary and quaternary materials. Nanoparticle material includes, but is not restricted to: Al₂S₃, Al₂Se₃, Al₂Te₃, Ga₂S₃, Ga₂Se₃, In₂S₃, In₂Se₃, Ga₂Te₃, In₂Te₃.
IV compounds including elements from group 14 (IV): Si, Ge, SiC, SiGe.
IV-VI compounds including a first element from group 14 (IV) of the periodic table and a second element from group 16 (VI) of the periodic table, as well as ternary and quaternary materials including, but not restricted to: PbS, PbSe, PbTe, SnSeS, SnSeTe, SnSTe, PbSeS, PbSeTe, PbSTe, SnPbSe, SnPbTe, SnPbSeTe, SnPbSTe.

The shell layer(s) grown on the nanoparticle core may include any one or more of the following materials:
IIA-VIB (2-16) material, incorporating a first element from group 2 of the periodic table and a second element from group 16 of the periodic table, and also including ternary and quaternary materials and doped materials. Nanoparticle material includes, but is not restricted to: MgS, MgSe, MgTe, CaS, CaSe, CaTe, SrS, SrSe, SrTe.
IIB-VIB (12-16) material incorporating a first element from group 12 of the periodic table and a second element from group 16 of the periodic table, and also including ternary and quaternary materials and doped materials. Nanoparticle material includes, but is not restricted to: ZnS, ZnSe, ZnTe, CdS, CdSe, CdTe, HgS, HgSe, HgTe.
II-V material incorporating a first element from group 12 of the periodic table and a second element from group 15 of the periodic table, and also including ternary and quaternary materials and doped materials. Nanoparticle material includes, but is not restricted to: Zn₃P₂, Zn₃As₂, Cd₃P₂, Cd₃As₂, Cd₃N₂, Zn₃N₂.
III-V material incorporating a first element from group 13 of the periodic table and a second element from group 15 of the periodic table, and also including ternary and quaternary materials and doped materials. Nanoparticle material includes, but is not restricted to: BP, AlP, AlAs, AlSb, GaN, GaP, GaAs, GaSb, InN, InP, InAs, InSb, AlN, BN.
III-IV material incorporating a first element from group 13 of the periodic table and a second element from group 14 of the periodic table, and also including ternary and quaternary materials and doped materials. Nanoparticle material includes, but is not restricted to: B₄C, Al₄C₃, Ga₄C.
III-VI material incorporating a first element from group 13 of the periodic table and a second element from group 16 of the periodic table, and also including ternary and quaternary materials. Nanoparticle material includes, but is not restricted to: Al₂S₃, Al₂Se₃, Al₂Te₃, Ga₂S₃, Ga₂Se₃, In₂S₃, In₂Se₃, Ga₂Te₃, In₂Te₃.
IV-VI material incorporating a first element from group 14 of the periodic table and a second element from group 16 of the periodic table, and also including ternary and quaternary materials and doped materials. Nanoparticle material includes, but is not restricted to: PbS, PbSe, PbTe, Sb₂Te₃, SnS, SnSe, SnTe.

Nanoparticle material incorporating a first element from any group in the d-block of the periodic table, and a second element from group 16 of the periodic table, and also including ternary and quaternary materials and doped materials. Nanoparticle material includes, but is not restricted to: NiS, CrS, CuInS₂, CuInSe₂, CuGaS₂, CuGaSe₂.

The coordination around the atoms on the surface of any core, core-shell or core-multishell, doped or graded nanoparticle is incomplete and the non-fully coordinated atoms have dangling bonds which make them highly reactive and may lead to particle agglomeration. This problem is overcome by passivating (capping) the "bare" surface atoms with protecting organic groups.

The outermost layer (capping agent) of organic material or sheath material helps to inhibit particle-particle aggregation, further protecting the nanoparticles from their surrounding electronic and chemical environments. In many cases, the capping agent is the solvent in which the nanoparticle preparation is undertaken, and consists of a Lewis base compound, or a Lewis base compound diluted in an inert solvent such as a hydrocarbon. There is a lone pair of electrons on the Lewis base capping agent that is capable of a donor-type coordination to the surface of the nanoparticle and include mono- or multi-dentate ligands such as phosphines (trioctylphosphine, triphenylphosphine, t-butylphosphine, etc.), phosphine oxides (trioctylphosphine oxide, triphenylphosphine oxide, etc.), alkyl phosphonic acids, alkyl-amines (octadecylamine, hexadecylamine, octylamine, etc.), aryl-amines, pyridines, long chain fatty acids (myristic acid, oleic acid, undecylenic acid, etc.) and thiophenes but is, as one skilled in the art will know, not restricted to these materials.

The outermost layer (capping agent) of a QD may also include a coordinated ligand with additional functional groups that may be used as chemical linkage to other inorganic, organic or biological material, whereby the functional group is pointing away from the QD surface and is available to bond/react/interact with other available molecules, such as amines, alcohols, carboxylic acids, esters, acid chlorides, anhydrides, ethers, alkyl halides, amides, alkenes, alkanes, alkynes, allenes, amino acids, azide groups, etc. but is, as one skilled in the art will know, not limited to these functionalised molecules. The outermost layer (capping agent) of a QD may also include of a coordinated ligand with a functional group that is polymerisable and may be used to form a polymer layer around the particle.

The outermost layer (capping agent) may also include organic units that are directly bonded to the outermost inorganic layer such as via an S-S bond between the inorganic surface (ZnS) and a thiol capping molecule. These may also possess additional functional group(s), not bonded to the surface of the particle, which may be used to form a polymer around the particle, or for further reaction/interaction/chemical linkage.

The LEDs described herein may be fabricated with "bare" QDs embedded directly into the LED encapsulant, or more preferably, they may be incorporated into microbeads prior to their embedment into the LED encapsulant; the QD microbeads exhibit superior robustness and longer lifetimes than bare QDs, and are more stable to the mechanical and thermal processing protocols of LED fabrication. The terms "beads" and "microbeads" are used interchangeably herein. Polymer beads are discussed herein, but the beads may also be of different materials, such as sol-gels, silica, or glass, as described in co-owned patent application, serial no. 12/622,012, filed November 19, 2009, (Pub. No.: 2010/0123155) the contents of which are incorporated herein by reference.

By incorporating the QD material into polymer microbeads, the nanoparticles become more resistant to air, moisture and photo-oxidation, opening up the possibility for processing in air that would vastly reduce the manufacturing cost. The bead size may be tuned from 20 nm to 0.5 mm, enabling control over the ink viscosity without changing the inherent optical properties of the QDs. The viscosity dictates how the QD bead ink flows through a mesh, dries, and adheres to a substrate, so thinners are not required to alter the viscosity, reducing the cost of the ink formulation. By incorporating the QDs into microbeads, the detrimental effect of particle agglomeration on the optical performance of bare encapsulated QDs is eliminated. QD beads provide an effective means of colour mixing.

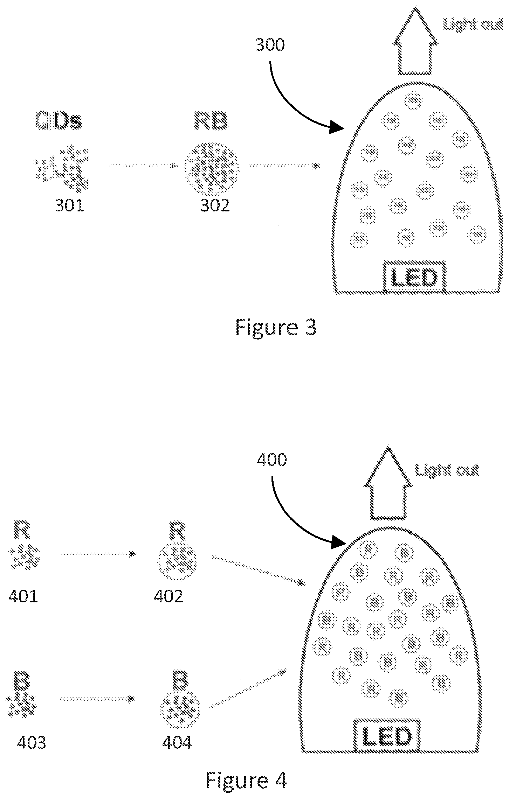

Figure 3 illustrates an LED device 300 made using a mixture of red and blue QDs 301 mixed together in beads 302. Figure 4 illustrates an alternative embodiment of an LED device 400 wherein red QDs 401 are contained in bead 402 and blue QDs 403 are contained in bead 404.

One such method for incorporating QDs into microbeads involves growing the polymer bead around the QDs. A second method incorporates QDs into pre-existing microbeads.

With regard to the first option, by way of example, hexadecylamine-capped CdSe-based semiconductor nanoparticles may be treated with at least one, more preferably two or more polymerisable ligands (optionally one ligand in excess) resulting in the displacement of at least some of the hexadecylamine capping layer with the polymerisable ligand(s). The displacement of the capping layer with the polymerisable ligand(s) may be accomplished by selecting a polymerisable ligand or ligands with structures similar to that of trioctylphosphine oxide (TOPO), which is a ligand with a known and very high affinity for CdSe-based nanoparticles. It will be appreciated that this basic methodology may be applied to other nanoparticle/ligand pairs to achieve a similar effect. That is, for any particular type of nanoparticle (material and/or size), it is possible to select one or more appropriate polymerisable surface binding ligands by choosing polymerisable ligands comprising a structural motif which is analogous in some way (e.g. has a similar physical and/or chemical structure) to the structure of a known surface binding ligand. Once the nanoparticles have been surface-modified in this way, they may then be added to a monomer component of a number of microscale polymerisation reactions to form a variety of QD-containing resins and beads. Another option is the polymerisation of one or more polymerisable monomers from which the optically transparent medium is to be formed in the presence of at least a portion of the semiconductor nanoparticles to be incorporated into the optically transparent medium. The resulting materials incorporate the QDs covalently and appear highly coloured even after prolonged periods of Soxhlet extraction.

Examples of polymerisation methods that may be used to construct QD-containing beads include, but are not restricted to, suspension, dispersion, emulsion, living, anionic, cationic, RAFT, ATRP, bulk, ring-closing metathesis and ring-opening metathesis, Initiation of the polymerisation reaction may be induced by any suitable method that causes the monomers to react with one another, such as by the use of free radicals, light, ultrasound, cations, anions, or heat. A preferred method is suspension polymerisation, involving thermal curing of one or more polymerisable monomers from which the optically transparent medium is to be formed. Said polymerisable monomers preferably comprise methyl (meth)acrylate, ethylene glycol dimethacrylate and vinyl acetate. This combination of monomers has been shown to exhibit excellent compatibility with existing commercially available LED encapsulants and has been used to fabricate a light emitting device exhibiting significantly improved performance compared to a device prepared using essentially prior art methodology. Other preferred polymerisable monomers are epoxy or polyepoxide monomers, which may be polymerised using any appropriate mechanism, such as curing with ultraviolet irradiation.

QD-containing microbeads may be produced by dispersing a population of QDs within a polymer matrix, curing the polymer and then grinding the resulting cured material. This is particularly suitable for use with polymers that become relatively hard and brittle after curing, such as many common epoxy or polyepoxide polymers (e.g. Optocast™ 3553 from Electronic Materials, Inc., USA).

QD-containing beads may be generated simply by adding QDs to the mixture of reagents used to construct the beads. In some instances, nascent QDs will be used as isolated from the reaction employed for their synthesis, and are thus generally coated with an inert outer organic ligand layer. In an alternative procedure, a ligand exchange process may be carried out prior to the bead-forming reaction. Here, one or more chemically reactive ligands (for example a ligand for the QDs that also contains a polymerizable moiety) are added in excess to a solution of nascent QDs coated in an inert outer organic layer. After an appropriate incubation time the QDs are isolated, for example by precipitation and subsequent centrifugation, washed and then incorporated into the mixture of reagents used in the bead forming reaction/process.

Both QD incorporation strategies will result in statistically random incorporation of the QDs into the beads and thus the polymerisation reaction will result in beads containing statistically similar amounts of the QDs. It will be obvious to one skilled in the art that bead size may be controlled by the choice of polymerisation reaction used to construct the beads, and additionally once a polymerisation method has been selected bead size may also be controlled by selecting appropriate reaction conditions, e.g. by stirring the reaction mixture more quickly in a suspension polymerisation reaction to generate smaller beads. Moreover, the shape of the beads may be readily controlled by choice of procedure in conjunction with whether or not the reaction is carried out in a mould. The composition of the beads may be altered by changing the composition of the monomer mixture from which the beads are constructed. Similarly, the beads may also be cross-linked with varying amounts of one or more cross-linking agents (e.g. divinyl benzene). If beads are constructed with a high degree of cross-linking, e.g. greater than 5 mol % cross-linker, it may be desirable to incorporate a porogen (e.g. toluene or cyclohexane) during the bead-forming reaction. The use of a porogen in such a way leaves permanent pores within the matrix constituting each bead. These pores may be sufficiently large to allow the ingress of QDs into the bead.

QDs may also be incorporated in beads using reverse emulsion-based techniques. The QDs may be mixed with precursor(s) to the optically transparent coating material and then introduced into a stable reverse emulsion containing, for example, an organic solvent and a suitable salt. Following agitation the precursors form microbeads encompassing the QDs, which may then be collected using any appropriate method, such as centrifugation. If desired, one or more additional surface layers or shells of the same or a different optically transparent material may be added prior to isolation of the QD-containing beads by addition of further quantities of the requisite shell layer precursor material(s).

In respect of the second option for incorporating QDs into beads, the QDs may be immobilised in polymer beads through physical entrapment. For example, a solution of QDs in a suitable solvent (e.g. an organic solvent) may be incubated with a sample of polymer beads. Removal of the solvent using any appropriate method results in the QDs becoming immobilised within the matrix of the polymer beads. The QDs remain immobilised in the beads unless the sample is resuspended in a solvent (e.g. organic solvent) in which the QDs are freely soluble. Optionally, at this stage the outside of the beads may be sealed. Alternatively, at least a portion of the QDs may be physically attached to prefabricated polymer beads. Said attachment may be achieved by immobilisation of the portion of the semiconductor nanoparticles within the polymer matrix of the prefabricated polymeric beads or by chemical, covalent, ionic, or physical connection between the portion of semiconductor nanoparticles and the prefabricated polymeric beads. Examples of prefabricated polymeric beads comprise polystyrene, polydivinyl benzene and a polythiol.

QDs may be irreversibly incorporated into prefabricated beads in a number of ways, e.g. chemical, covalent, ionic, physical (e.g. by entrapment) or any other form of interaction. If prefabricated beads are to be used for the incorporation of QDs, the solvent-accessible surfaces of the bead may be chemically inert (e.g. polystyrene) or alternatively they may be chemically reactive/functionalised (e.g. Merrifield's Resin). The chemical functionality may be introduced during the construction of the bead, for example by the incorporation of a chemically functionalised monomer, or, alternatively, chemical functionality may be introduced in a post bead construction treatment, for example by conducting a chloromethylation reaction. Additionally, chemical functionality may be introduced by a post-bead construction polymeric graft or other similar process whereby chemically reactive polymer(s) are attached to the outer layers/accessible surfaces of the bead. More than one such post-construction derivation process may be carried out to introduce chemical functionality onto/into the bead.

As with QD incorporation into beads during the bead forming reaction, i.e. the first option described above, the pre-fabricated beads may be of any shape, size and composition, may have any degree of cross-linker and may contain permanent pores if constructed in the presence of a porogen. QDs may be imbibed into the beads by incubating a solution of QDs in an organic solvent and adding this solvent to the beads. The solvent must be capable of wetting the beads and, in the case of lightly cross-linked beads, preferably 0-10 % cross-linked and most preferably 0-2 % cross-linked, the solvent should cause the polymer matrix to swell in addition to solvating the QDs. Once the QD-containing solvent has been incubated with the beads, it is removed, for example by heating the mixture and causing the solvent to evaporate, and the QDs become embedded in the polymer matrix constituting the bead or alternatively by the addition of a second solvent in which the QDs are not readily soluble but which mixes with the first solvent causing the QDs to precipitate within the polymer matrix constituting the beads. Immobilisation may be reversible if the bead is not chemically reactive, or else if the bead is chemically reactive the QDs may be held permanently within the polymer matrix by chemical, covalent, ionic, or any other form of interaction.

Optically transparent media that are sol-gels and glasses, intended to incorporate QDs, may be formed in an analogous fashion to the method used to incorporate QDs into beads during the bead-forming process as described above. For example, a single type of QD (e.g. one colour) may be added to the reaction mixture used to produce the sol-gel or glass. Alternatively, two or more types of QD (e.g. two or more colours) may be added to the reaction mixture used to produce the sol-gel or glass. The sol-gels and glasses produced by these procedures may have any shape, morphology or 3-dimensional structure. For example, the particles may be spherical, disc-like, rod-like, ovoid, cubic, rectangular, or any of many other possible configurations.

By incorporating QDs into beads in the presence of materials that act as stability-enhancing additives, and optionally providing the beads with a protective surface coating, migration of deleterious species, such as moisture, oxygen and/or free radicals, is eliminated or at least reduced, with the result of enhancing the physical, chemical and/or photo-stability of the semiconductor nanoparticles.

An additive may be combined with "bare" semiconductor nanoparticles and precursors at the initial stages of the production process of the beads. Alternatively, or additionally, an additive may be added after the semiconductor nanoparticles have been entrapped within the beads.

The additives that may be added singly or in any desirable combination during the bead formation process may be grouped according to their intended function, as follows:
Mechanical sealing: fumed silica (e.g. Cab-O-Sil™), ZnO, TiO2, ZrO, Mg stearate, Zn stearate, all used as a filler to provide mechanical sealing and/or reduce porosity.

Capping agents: tetradecyl phosphonic acid (TDPA), oleic acid, stearic acid, polyunsaturated fatty acids, sorbic acid, Zn methacrylate, Mg stearate, Zn stearate, isopropyl myristate. Some of these have multiple functionalities and may act as capping agents, free radical scavengers and/or reducing agents.

Reducing agents: ascorbic acid palmitate, alpha tocopherol (vitamin E), octane thiol, butylated hydroxyanisole (BHA), butylated hydroxytoluene (BHT), gallate esters (propyl, lauryl, octyl, etc.), a metabisulfite (e.g. the sodium or potassium salt).

Free radical scavengers: benzophenones.

Hydride reactive agents: 1,4-butandiol, 2-hydroxyethyl methacrylate, allyl methacrylate, 1,6-heptadiene-4-ol, 1,7-octadiene, and 1,4-butadiene.

The selection of the additive(s) for a particular application will depend upon the nature of the semiconductor nanoparticle material (e.g. how sensitive the nanoparticle material is to physical, chemical and/or photo-induced degradation), the nature of the primary matrix material (e.g. how porous it is to potentially deleterious species, such as free-radicals, oxygen, moisture, etc.), the intended function of the final material or device which will contain the primary particles (e.g. the operating conditions of the material or device), and the process conditions required to fabricate the said final material or device. With this in mind, one or more appropriate additives may be selected from the above five lists to suit any desirable semiconductor nanoparticle application.

Once the QDs are incorporated into the beads, the formed QD-beads may be further coated with a suitable material to provide each bead with a protective barrier to prevent the passage or diffusion of potentially deleterious species, e.g. oxygen, moisture or free radicals from the external environment, through the bead material to the semiconductor nanoparticles. As a result, the semiconductor nanoparticles are less sensitive to their surrounding environment and the various processing conditions typically required to utilise the nanoparticles in applications such as the fabrication of LEDs.

The coating is preferably a barrier to the passage of oxygen or any type of oxidising agent through the bead material. The coating may be a barrier to the passage of free radical species and/or is preferably a moisture barrier so that moisture in the environment surrounding the beads cannot contact the semiconductor nanoparticles incorporated within the beads.

The coating may provide a layer of material on a surface of the bead of any desirable thickness, provided it affords the required level of protection. The surface layer coating may be around 1 to 10 nm thick, up to around 400 to 500 nm thick, or more. Preferred layer thicknesses are in the range 1 nm to 200 nm, more preferably around 5 nm to 100 nm.

The coating may comprise an inorganic material, such as a dielectric (insulator), a metal oxide, a metal nitride or a silica-based material (e.g. a glass).

The metal oxide may be a single metal oxide (*i.e*. oxide ions combined with a single type of metal ion, e.g. Al₂O₃), or may be a mixed metal oxide (*i.e.* oxide ions combined with two or more types of metal ion, *e.g.* SrTiO₃). The metal ion(s) of the (mixed) metal oxide may be selected from any suitable group of the periodic table, such as group 2, 13, 14 or 15, or may be a transition metal, d-block metal, or lanthanide metal.

Preferred metal oxides are selected from the group consisting of Al₂O₃, B₂O₃, Co₂O₃, Cr₂O₃, CuO, Fe₂O₃, Ga₂O₃, HfO₂, In₂O₃, MgO, Nb₂O₅, NiO, SiO₂, SnO₂, Ta₂O₅, TiO₂, ZrO₂, SC₂O₃, Y₂O₃, GeO₂, La₂O₃, CeO₂, PrOₓ (x = appropriate integer), Nd₂O₃, Sm₂O₃, EuO_{y} (y = appropriate integer), Gd₂O₃, Dy₂O₃, Ho₂O₃, Er₂O₃, Tm₂O₃, Yb₂O₃, Lu₂O₃, SrTiO₃, BaTiO₃, PbTiO₃, PbZrO₃, BiₘTiₙO (m, n = appropriate integer), BiₐSi_{b}O (a, b = appropriate integer), SrTa₂O₆, SrBi₂Ta₂O₉, YScO₃, LaAlO₃, NdAlO₃, GdScO₃, LaScO₃, LaLuO₃, Er₃Ga₅O₁₃.

Preferred metal nitrides may be selected from the group consisting of BN, AlN, GaN, InN, Zr₃N₄, Cu₂N, Hf₃N₄, SiNc (c = appropriate integer), TiN, Ta₃N₅, Ti-Si-N, Ti-Al-N, TaN, NbN, MoN, WNd (d = appropriate integer), WNeCf (e, f = appropriate integer).

The inorganic coating may comprise silica in any appropriate crystalline form.

The coating may incorporate an inorganic material in combination with an organic or polymeric material, e.g. an inorganic/polymer hybrid, such as a silica-acrylate hybrid material.

The coating may comprise a polymeric material which may be a saturated or unsaturated hydrocarbon polymer, or may incorporate one or more heteroatoms (e.g. O, S, N, halo) or heteroatom-containing functional groups (e.g. carbonyl, cyano, ether, epoxide, amide, etc.).

Examples of preferred polymeric coating materials include acrylate polymers (e.g. polymethyl(meth)acrylate, polybutylmethacrylate, polyoctylmethacrylate, alkylcyanoacryaltes, polyethyleneglycol dimethacrylate, polyvinylacetate, etc.), epoxides (e.g. EPOTEK 301 A and B Thermal curing epoxy, EPOTEK OG112-4 single-pot UV curing epoxy, or EX0135 A and B Thermal curing epoxy), polyamides, polyimides, polyesters, polycarbonates, polythioethers, polyacrylonitryls, polydienes, polystyrene polybutadiene copolymers (Kratons), pyrelenes, poly-para-xylylene (parylenes), polyetheretherketone (PEEK), polyvinylidene fluoride (PVDF), polydivinyl benzene, polyethylene, polypropylene, polyethylene terephthalate (PET), polyisobutylene (butyl rubber), polyisoprene, and cellulose derivatives (methyl cellulose, ethyl cellulose, hydroxypropylmethyl cellulose, hydroxypropylmethylcellulose phthalate, nitrocellulose), and combinations thereof.

### Device Architectures

The QD LED device architectures described herein serve only as examples, but the invention is not restricted to these. Any suitable device architecture incorporating QDs to emit at wavelengths to promote plant growth and development may be employed.

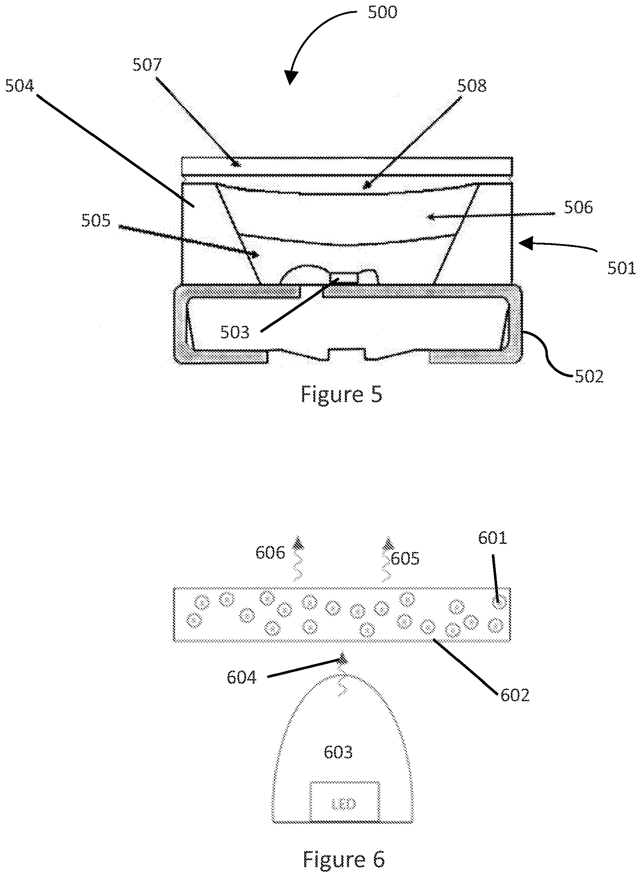

QD LED devices may be fabricated using the structure of the QD LED chip shown in Figure 5. Device 500 includes an LED assembly 501 mounted in a housing 502. The LED assembly includes a solid-state LED material 503, which is generally a UV or blue-emitting LED material, such as YAG. LED material 503 is contained within an LED package 504 and protected by an LED encapsulant material 505. An example of an LED encapsulant material 505 is silicone, although any encapsualnt material can be used. QD-containing resin 506 is disposed on top encapsualnt material 505. Exemplary resins, as described above, include polymer resins containing QDs dispersed therein. The QDs can be embedded in beads or can be "naked." In other embodiments, the QD-containing resin 506 may be disposed directly on LED material 503. In other words, the LED encapsulant material and the QD-resin may be a single layer, rather than two layers as illustrated in Figure 5. The QD-containing resin 506 is protected by a thin transparent material 507, such as glass, which can be sealed in place by adhesive 508, such as epoxy.

The embodiment illustrated in Figure 5 can be made as follows: In a nitrogen-filled glove box, the LED chip is first covered with a blank silicone resin to protect the chip from any damage from the acrylate resin in which the QDs are embedded. The silicone resin is cured on a hotplate. The LED cup is then filled with the QD-embedded acrylate resin, which is cured under UV light. The LED is then encapsulated using a thin layer of gas barrier, attached using a UV curing epoxy resin (e.g. Optocast™), and cured under UV light. A UV or blue solid state LED is fitted to the bottom of the LED chip to illuminate the QDs.

As an example of embodiments wherein QDs are contained in beads, silicone resin can be mixed with a small amount of a Pt catalyst. Then the QD beads are added to the silicone mixture and the mixture is transferred to LED packages. The packages are cured under a nitrogen atmosphere, then encapsulated under a thin layer of gas barrier, attached using a UV curing epoxy resin, as described above. The LEDs are cured under UV light.

Figure 6 illustrates a QD LED device having a remote phosphor architecture, i.e., an architecture in which the QD material is outside the LED package. As illustrated in Figure 6, a polymer film containing red QDs 601 is disposed on a substrate to provide a QD phosphor sheet 602, which is situated remote from LED package 603. As above, QDs 601 may be in beads or may be "naked." QDs 601 absorb blue light 604 produced by the LED in package 603 (in the case that package 603 contains a blue LED). Light emanating from the phosphor sheet 602 is a mixture of red light 605 emitted by the QDs 601 and blue light 606 transmitted through phosphor sheet 602.

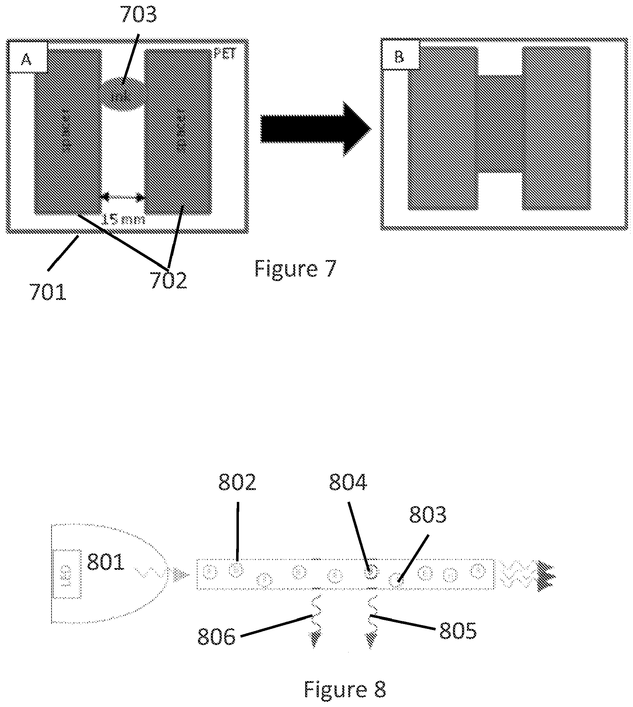

Figure 7 illustrates an embodiment of how the remote QD phosphor sheet used in the architecture illustrated in Figure 6 is prepared. QD ink is prepared by mixing a known quantity of QD solution with appropriate solvents and polymerisable materials. A PET sheet (or other suitable substrate) 701 of predetermined dimensions is cleaned with an air gun to remove dust particles and fitted with two Teflon spacers 702, ensuring that a constant gap is left between the spacers. Under a nitrogen atmosphere, the ink is drop 703 casted on the region between the spacers of the substrate and the ink is distributed uniformly between the spacers. The substrate is placed on a preheated hotplate to remove any solvent, then allowed to dry. The encapsulated QD phosphor is illuminate from behind using a series of UV or blue solid state LEDs. These excite the QDs in the phosphor layer, which emit at a specific wavelength that is selected to optimise photosynthetic growth, while some UV or blue light from the SS LEDs is still transmitted through the glass. The emission of the QD phosphor and the UV or blue LEDs may be tuned to match the absorption spectra of chlorophyll and the accessory pigments in specific organisms, thus optimising photosynthesis.

Figure 8 illustrates an embodiment wherein a solid state LED 801 is located at the side of the QD phosphor sheet 802. The remote QD phosphor sheet 802 is prepared as described above (see Figure 7). In the embodiment illustrated in Figure 8, QD phosphor sheet 802 includes red-emitting 803 and blue-emitting 804 QDs. The encapsulated QD phosphor sheet 802 is illuminated from the side by a solid state UV or blue-emitting LED 801. The down-converted red 805 and blue 806 emission of the QDs is emitted perpendicular to the incident LED emission.

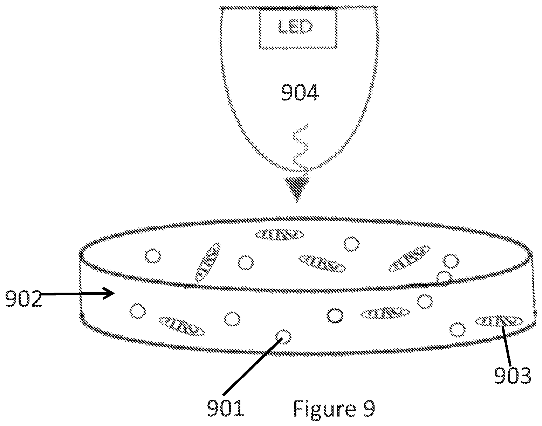

Figure 9 illustrates an embodiment wherein water-soluble QD microbeads 901 are mixed into an agar preparation 902, which is used as a growth culture for photosynthetic bacteria 903. The QD-agar preparation is placed in a transparent container and illuminated externally using UV or blue LED light (904). The QDs in the agar down-convert the emission from the LED, while some of the primary LED light is still transmitted through the culture medium.

Figure 10 shows the overlap of emission from a QD LED chip with the absorbance wavelengths of chlorophyll a and chlorophyll b. A QD LED chip having a blue solid state LED as the primary light source and adapted with a red-emitting QD/silicone resin was fabricated according to the following procedure: Silicone resin was mixed with a small amount of a Pt catalyst, then red InP/ZnS QD beads (20 ODs per 10 mmol solution in toluene) were added and the mixture was transferred to an LED case. The LED was cured under a nitrogen atmosphere. The QD LED was illuminated by a 22 mW blue solid state LED. Blue emission was seen around 455 nm from the solid state LED. The red QD material emitted with PLₘₐₓ = 648 nm and FWHM = 59 nm. The relative intensity of the blue (LED) to red (QD phosphor) light was 1:0.45.

A QD LED chip comprising a blue solid state LED as the primary light source and a red quantum dot silicone resin was fabricated according to the following procedure: Red InP/ZnS QD beads were diluted to 10 ODs/10 mmol in toluene. Silicone resin was mixed with a small amount of a Pt catalyst, then the QD beads were added and the mixture is transferred to an LED case. The LED was cured under a nitrogen atmosphere. The QD LED was illuminated by a 22 mW blue solid state LED. Blue emission was seen around 455 nm from the solid state LED. The red QD material emitted with PLmax = 646 nm and FWHM = 60 nm. The relative intensity of the blue (LED) to red (QD phosphor) light was 1:0.27.

A QD LED chip comprising a blue solid state LED as the primary light source and a red quantum dot silicone resin, with an emission spectrum that is well matched to the absorption spectrum of chlorophyll b, was fabricated using CdSe/CdS/CdZnS/ZnS core-multishell QDs illuminated by a blue solid state LED backlight emitting at 446 nm. The QD PLmax of 625 nm is well-matched to the red absorption maximum of chlorophyll b, with a narrow FWHM of 35 nm, as shown in Figure 11. The relative blue and red peak intensities may be matched to those of the absorption spectrum of chlorophyll b by altering the QD concentration.

### Lighting Arrangements

Because QD LED chips can vary from very small LED cups to large printed QD LED phosphor sheets encased in glass, many different lighting arrangements are possible. The QD LED chips described herein are suitable for both small, light, portable devices and large permanent fixtures. For example, an LED backlight combined with a QD phosphor sheet, as illustrated in Figure 6, can be used to make a portable device that can be used to promote grass growth for reseeding areas of turf. The device emits both blue and red light. Using a portable, retractable lighting fixture, the light may be focussed on small areas to accelerate grass growth and may be quickly and easily transported.

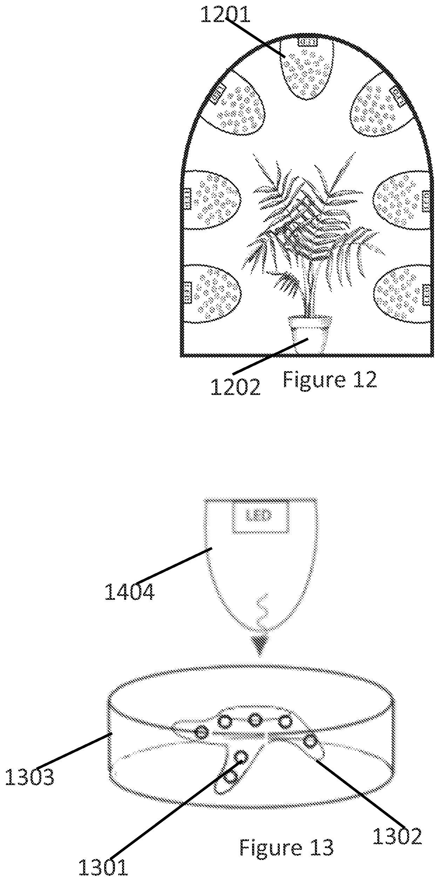

Figure 12 illustrates an arched or caged framework fitted QD LEDs 1201, as shown in Figures 3 or 4, in which red and blue QDs are illuminated by a UV solid state LED. One or more plants 1202 may be placed inside the framework, which provides illumination from many directions to not only promote photosynthesis, but also uniform growth. The framework may be constructed from any suitable material, though a reflective material may be advantageous.

Figure 13 illustrates a lighting arrangement wherein a red QD phosphor is printed on a substrate 1302 with finger-like projections that is immersed in a photobioreactor 1303. The QD phosphor is illuminated, either from above and/or below, with blue solid state LEDs. Secondary emission from the QD phosphor is projected into the photobioreactor. Alternatively (or in additionally), the photobioreactor itself can be fabricated using a transparent material printed with red QD ink. When the photobioreactor is illuminated from outside the photobioreactor by blue (or UV) solid state LEDs, both blue (from the LED) and red (from the QD phosphor) light is emitted into the photobioreactor.

Although particular embodiments of the invention have been shown and described, they are not intended to limit what this patent covers. One skilled in the art will understand that that various changes and modifications may be made without departing from the scope of the present invention as literally and equivalently covered by the following claims.

## Claims

1. An apparatus for promoting growth in a photosynthetic organism, the apparatus comprising:
a light emitting device comprising a primary light-emitting element that emits light at a first wavelength; and
a quantum dot (QD) population positioned to absorb a portion of light emitted by the primary light emitting element and to emit light at a second wavelength; wherein
the primary light-emitting element is a solid state LED; wherein
the first wavelength is in the UV or blue region of the electromagnetic spectrum; wherein
the second wavelength corresponds to a wavelength within a peak in an absorption spectrum of a photosynthetic pigment of the photosynthetic organism; wherein
the quantum dots are incorporated into beads; wherein
the beads comprise a coating disposed upon the surface of the beads, the coating comprising a dielectric, a metal oxide, a metal nitride, a silica-based material, a polymeric material or an inorganic/polymer hybrid material.

2. The apparatus of claim 1, wherein each bead comprises a material selected from polymers, sol-gels, silica gel, and glass.

3. The apparatus of claim 1, wherein the QD population is contained within an LED encapsulant that is contained within an LED package, optionally wherein the encapsulant is a silicone or an acrylate.

4. The apparatus of claim 1, wherein the QD population is provided on a substrate that is remote from the primary light-emitting element.

5. The apparatus of claim 1, wherein:
i) the metal oxide of the coating is selected from the group consisting of Al₂O₃, B₂O₃, Co₂O₃, Cr₂O₃, CuO, Fe₂G₃, Ga₂O₃, HfO₂, In₂O₃, MgO, Nb₂O₅, NiO, SiO₂, SnO₂, Ta₂O₅, TiO₂, ZrO₂, Sc₂O₃, Y₂O₃, GeO₂, La₂O₃, CeO₂, PrOₓ, Nd₂O₃, Sm₂O₃, EuO_{y}, Gd₂O₃, Dy₂O₃, Ho₂O₃, Er₂O₃, Tm₂O₃, Yb₂O₃, Lu₂O₃, SrTiO₃, BaTiO₃, PbTiO₃ PbZrO₃, BiₘTiₙO, BiₐSi_{b}O, SrTa₂O₆, SrBi₂Ta₂O₉, YScO₃, LaAlO₃, NdAlO₃, GdScO₃, LaScO₃, LaLuO₃, Er₃Ga₅O₁₃, wherein a, b, m, n, x and y are integers;
ii) the metal nitride of the coating is selected from the group consisting of BN, AIN, GaN, InN, Zr3N4, Cu2N, Hf3N4, SiNc, TiN, Ta3N5, Ti-Si-N, Ti-Al-N, TaN, NbN, MoN, WNd, WNeCf wherein c, d, e and f are integers;
iii) the polymeric material of the coating is selected from acrylate polymers, epoxides, polyamides, polyimides, polyesters, polycarbonates, polythioethers, polyacrylonitryls, polydienes, polystyrene polybutadiene copolymers, pyrelenes, poly-para-xylylene, polyetheretherketone (PEEK), polyvinylidene fluoride, polydivinyl benzene, polyethylene, polypropylene, polyethylene terephthalate (PET), polyisobutylene, polyisoprene, and cellulose derivatives, and combinations thereof.

6. The apparatus of claim 1, wherein the QD population does not contain cadmium, optionally wherein the QD population comprises QDs having indium phosphide cores and shells of zinc sulphide or zinc selenide.

7. The apparatus of claim 1, wherein the photosynthetic pigment is chlorophyll.

8. The apparatus of claim 1, wherein the apparatus comprises a photobioreactor.

9. The apparatus of claim 8, wherein the QD population is disposed: (i) on walls of the photobioreactor; or (ii) on a substrate that is immersed within the photobioreactor.

10. The apparatus of claim 8, wherein the photobioreactor is for growing algae or bacteria.

11. The apparatus of claim 8, wherein the photobioreactor is a transparent container.

12. The apparatus of claim 8, wherein a culture medium is contained within the photobioreactor.

13. The apparatus of claim 12, wherein the culture medium is used as a growth culture for photosynthetic bacteria and algae.

14. The apparatus of claim 12, wherein the culture medium comprises agar.

## Patentansprüche

1. Einrichtung zum Fördern des Wachstums in einem fotosynthetischen Organismus, wobei die Einrichtung umfasst:
eine lichtemittierende Vorrichtung, die ein primäres lichtemittierendes Element umfasst, das Licht bei einer ersten Wellenlänge emittiert;
und eine Quantenpunktpopulation (QD-Population), die positioniert ist, einen Teil von Licht, das durch das primäre lichtemittierende Element emittiert wird, zu absorbieren und Licht bei einer zweiten Wellenlänge zu emittieren;
wobei das primäre lichtemittierende Element eine Festkörper-LED ist;
wobei die erste Wellenlänge in dem UV- oder dem Blaubereich des elektromagnetischen Spektrums liegt;
wobei die zweite Wellenlänge einer Wellenlänge innerhalb einer Spitze in einem Absorptionsspektrum eines fotosynthetischen Pigments des fotosynthetischen Organismus entspricht;
wobei die Quantenpunkte in Kügelchen integriert sind;
wobei die Kügelchen eine auf der Oberfläche der Kügelchen angeordnete Beschichtung umfassen, wobei die Beschichtung ein Dielektrikum, ein Metalloxid, ein Metallnitrid, ein Material auf Siliziumbasis, ein Polymermaterial oder ein anorganisches/polymeres Hybridmaterial umfasst.

2. Einrichtung nach Anspruch 1, wobei jedes Kügelchen ein Material umfasst, das aus Polymeren, Sol-Gel, Kieselgel und Glas ausgewählt ist.

3. Einrichtung nach Anspruch 1, wobei die QD-Population in einem LED-Einkapselungsmittel enthalten ist, das in einem LED-Package enthalten ist, wobei das Einkapselungsmittel gegebenenfalls ein Silikon oder ein Acrylat ist.

4. Einrichtung nach Anspruch 1, wobei die QD-Population auf einem Substrat bereitgestellt ist, das von dem primären lichtemittierenden Element entfernt ist.

5. Einrichtung nach Anspruch 1, wobei:
i) das Metalloxid der Beschichtung aus der Gruppe ausgewählt ist, die aus Al₂O₃, B₂O₃, Co₂O₃, Cr₂O₃, CuO, Fe₂G₃, Ga₂O₃, HfO₂, In₂O₃, MgO, Nb₂O₅, NiO, SiO₂, SnO₂, Ta₂O₅, TiO₂, ZrO₂, SC₂O₃, Y₂O₃, GeO₂, La₂O₃, CeO₂, PrOₓ, Nd₂O₃, Sm₂O₃, EuO_{y}, Gd₂O₃, Dy₂O₃, Ho₂O₃, Er₂O₃, Tm₂O₃, Yb₂O₃, Lu₂O₃, SrTiO₃, BaTiO₃, PbTiO₃, PbZrO₃, BiₘTiₙO, BiₐSi_{b}O, SrTa₂O₆, SrBi₂Ta₂O₉, YScO₃, LaAlO₃, NdAlO₃, GdScO₃, LaScO₃, LaLuO₃, Er₃Ga₅O₁₃ besteht, wobei a, b, m, n, x und y ganze Zahlen sind;
ii) das Metallnitrid der Beschichtung aus der Gruppe ausgewählt ist, die aus BN, AlN, GaN, InN, Zr3N4, Cu2N, Hf3N4, SiNc, TiN, Ta3N5, Ti-Si-N, Ti-Al-N, TaN, NbN, MoN, WNd, WNeCf besteht, wobei c, d, e und f ganze Zahlen sind;
iii) das Polymermaterial der Beschichtung aus Acrylatpolymeren, Epoxiden, Polyamiden, Polyimiden, Polyestern, Polycarbonaten, Polythioethern, Polyacrylnitrilen, Polydienen, Polystyrol-Polybutadien-Copolymeren, Perylenen, Poly-para-xylol, Polyetheretherketon (PEEK), Polyvinylidenfluorid, Polydivinylbenzol, Polyethylen, Polypropylen, Polyethylen-Terephtalat (PET), Polyisobutylen, Polyisopren und Zellulosederivaten und Kombinationen davon ausgewählt ist.

6. Einrichtung nach Anspruch 1, wobei die QD-Population kein Cadmium enthält, wobei die QD-Population gegebenenfalls QDs umfasst, die Indiumphosphidkerne und Hüllen aus Zinksulfid oder Zinkselenid aufweisen.

7. Einrichtung nach Anspruch 1, wobei das fotosynthetische Pigment Chlorophyll ist.

8. Einrichtung nach Anspruch 1, wobei die Einrichtung einen Photobioreaktor umfasst.

9. Einrichtung nach Anspruch 8, wobei die QD Population (i) an Wänden des Photobioreaktors oder (ii) auf einem Substrat, das in den Photobioreaktor eingetaucht ist, angeordnet ist.

10. Einrichtung nach Anspruch 8, wobei der Photobioreaktor dem Züchten von Algen oder Bakterien dient.

11. Einrichtung nach Anspruch 8, wobei der Photobioreaktor ein transparenter Behälter ist.

12. Einrichtung nach Anspruch 8, wobei in dem Photobioreaktor ein Kulturmedium enthalten ist.

13. Einrichtung nach Anspruch 12, wobei das Kulturmedium als eine Wachstumskultur für fotosynthetische Bakterien und Algen verwendet wird.

14. Einrichtung nach Anspruch 12, wobei das Kulturmedium Agar umfasst.

## Revendications

1. Appareil permettant d'augmenter la croissance dans un organisme photosynthétique, l'appareil comprenant :
un dispositif électroluminescent comprenant un élément électroluminescent primaire, qui émet de la lumière à une première longueur d'onde ; et
une population à points quantiques (QD) positionnée de manière à absorber une partie de la lumière, émise par l'élément électroluminescent primaire, et à émettre de la lumière à une seconde longueur d'onde ;
dans lequel l'élément électroluminescent primaire est une LED à l'état solide ;
dans lequel la première longueur d'onde est dans la région des UV ou des bleus du spectre électromagnétique ;
dans lequel la seconde longueur d'ondes correspond à une longueur d'ondes dans un pic dans un spectre d'absorption d'un pigment photosynthétique de l'organisme photosynthétique ;
dans lequel les points quantiques sont incorporés dans des perles ;
dans lequel les perles comprennent un revêtement disposé sur la surface des perles, le revêtement comprenant un matériau diélectrique, un oxyde métallique, un nitrure métallique, un matériau à base de silice, un matériau polymère ou un matériau hybride inorganique/polymère.

2. Appareil selon la revendication 1, dans lequel chaque perle comprend un matériau choisi à partir de polymères, de sol-gels, de gel de silice et de verre.

3. Appareil selon la revendication 1, dans lequel la population QD est contenue dans un encapsulant à LED qui est contenu dans un emballage de LED, éventuellement dans lequel l'encapsulant est une silicone ou un acrylate.

4. Appareil selon la revendication 1, dans lequel la population QD est ménagée sur un substrat qui est éloigné de l'élément électroluminescent primaire.

5. Appareil selon la revendication 1, dans lequel :
i) l'oxyde métallique du revêtement est sélectionné dans le groupe constitué de Al₂O₃, B₂O₃, Co₂O₃, Cr₂O₃, CuO, Fe₂G₃, Ga₂O₃, HfO₂, In₂O₃, MgO, Nb₂O₅, NiO, SiO₂, SnO₂, Ta₂O₅, TiO₂, ZrO₂, SC₂O₃, Y₂O₃, GeO₂, La₂O₃, CeO₂, PrOₓ, Nd₂O₃, Sm₂O₃, EuO_{y}, Gd₂O₃, Dy₂O₃, Ho₂O₃, Er₂O₃, Tm₂O₃, Yb₂O₃, Lu₂O₃, SrTiO₃, BaTiO₃, PbTiO₃, PbZrO₃, BiₘTiₙO, BiₐSi_{b}O, SrTa₂O₆, SrBi₂Ta₂O₉, YScO₃, LaAlO₃, NdAlO₃, GdScO₃, LaScO₃, LaLuO₃, Er₃Ga₅O₁₃, dans lequel a, b, m, n, x et y sont des nombres entiers ;
ii) le nitrure métallique du revêtement est choisi dans le groupe constitué de BN, AlN, GaN, InN, Zr3N4, Cu2N, Hf3N4, SiNc, TiN, Ta3N5, Ti-Si-N, Ti-Al-N, TaN, NbN, MoN, WNd, WNeCf dans lequel c, d, e et f sont des nombres entiers.
iii) le matériau polymère du revêtement est choisi parmi des polymères d'acrylate, des époxydes, des polyamides, des polyimides, des polyesters, des polycarbonates, des polythioéthers, des polyacrylonitriles, des polydiènes, des copolymères polystyrène polybutadiène, des pyrélènes, des poly-para-xylylènes, des polyétheréthercétones (PEEK), du fluorure de polyvinylidène, du polydivinyl benzène, du polyéthylène, du polypropylène, du polyéthylène téréphtalate (PET), du polyisobutylène, du polyisoprène, et des dérivés de cellulose, et des combinaisons correspondantes.

6. Appareil selon la revendication 1, dans lequel la population QD ne contient pas de cadmium, éventuellement dans lequel la population QD comprend des QD présentant des noyaux en phosphure d'indium et des coques en sulfure de zinc ou en séléniure de zinc.

7. Appareil selon la revendication 1, dans lequel le pigment photosynthétique est la chlorophylle.

8. Appareil selon la revendication 1, dans lequel l'appareil comprend un photobioréacteur.

9. Appareil selon la revendication 8, dans lequel la population QD est disposée : (i) sur des parois du photobioréacteur ; ou (ii) sur un substrat qui est immergé dans le photobioréacteur.

10. Appareil selon la revendication 8, dans lequel le photobioréacteur est destiné à développer des algues ou bactéries.

11. Appareil selon la revendication 8, dans lequel le photobioréacteur est un récipient transparent.

12. Appareil selon la revendication 8, dans lequel un milieu de culture est contenu dans le photobioréacteur.

13. Appareil selon la revendication 12, dans lequel le milieu de culture est utilisé comme une culture de croissance pour des bactéries et des algues photosynthétiques.

14. Appareil selon la revendication 12, dans lequel le milieu de culture comprend de l'agar.
